(19) Europäisches Patentamt
European Patent Office
Office européen des brevets

(11) EP 1 460 123 A1

(12) **EUROPEAN PATENT APPLICATION**
published in accordance with Art. 158(3) EPC

(43) Date of publication:
**22.09.2004 Bulletin 2004/39**

(21) Application number: **02803561.6**

(22) Date of filing: **21.11.2002**

(51) Int Cl.$^7$: **C11B 9/00**, A01N 25/22, A61K 7/00, A61K 47/26, A61K 47/36, A23L 1/22

(86) International application number:
**PCT/JP2002/012196**

(87) International publication number:
**WO 2003/044143 (30.05.2003 Gazette 2003/22)**

(84) Designated Contracting States:
**AT BE BG CH CY CZ DE DK EE ES FI FR GB GR IE IT LI LU MC NL PT SE SK TR**

(30) Priority: **22.11.2001 JP 2001358562**
**19.04.2002 JP 2002118439**
**30.08.2002 JP 2002256070**

(83) **Declaration under Rule 28(4) EPC (expert solution)**

(71) Applicant: **Kabushiki Kaisha Hayashibara Seibutsu Kagaku Kenkyujo**
**Okayama-shi, Okayama 700-0907 (JP)**

(72) Inventors:
- **OKU, Kazuyuki,**
**K.K.Hayashib.Seibutsu Kag.Kenkyujo**
**Okayama-shi, Okayama 700-0907 (JP)**
- **KUBOTA, Michio,**
**K.K.Hayashib.Seibutsu Kag.Kenkyujo**
**Okayama-shi, Okayama 700-0907 (JP)**
- **FUKUDA, Shigeharu,**
**Hayashib.Seibutsu Kag.Kenkyujo**
**Okayama-shi, Okayama 700-0907 (JP)**
- **MIYAKE, Toshio,**
**K.K.Hayashib.Seibutsu Kag.Kenkyujo**
**Okayama-shi, Okayama 700-0907 (JP)**

(74) Representative: **Daniels, Jeffrey Nicholas et al**
**Page White & Farrer**
**54 Doughty Street**
**London WC1N 2LS (GB)**

# (54) METHOD OF SUSTAINING AROMA AND USE THEREOF

(57) The first object of the present invention is to provide a method for preparing an aroma-retaining material, which enables to increase the adsorbed aroma amount, retain the aroma for a long period of time, and impart stability. The second object of the present invention is to provide an aroma-retaining material, which can retain aroma for a long period of time and has satisfactory stability. The third object of the present invention is to provide a composition comprising the aroma-retaining material. The forth object of the present invention is to provide an aroma-retaining agent, which enables to stabilize an aromatic substance. The fifth object of the present invention is to provide a retained-releasing bactericide comprising the aroma-retaining material. The present invention solves the above objects by providing a method for retaining an aroma in such a manner of mixing an aromatic substance and a cyclic tetrasaccharide or the mixture of cyclic tetrasaccharide and its saccharide derivative(s), an aroma-retaining material obtainable by the method, a composition comprising the aroma-retaining material, an aroma-retaining agent comprising a cyclic tetrasaccharide or a mixture of a cyclic tetrasaccharide and its saccharide derivative(s) as effective ingredients, and a bactericide using the aroma-retaining material having a sustained-release property.

EP 1 460 123 A1

## Description

TECHNICAL FIELD

The present invention relates to a method for retaining aroma and use thereof, more particularly, to a method for retaining aroma, aroma-retaining materials obtainable by the method; compositions comprising the aroma-retaining materials, such as foods, cosmetics, pharmaceuticals and commodities; aroma-retaining agents; and bacteriostats and/or bactericides comprising the aroma-retaining agents having a sustained-release property.

BACKGROUND ART

The following methods have been known as methods for retaining aroma; those comprising a step of (i) allowing an aromatic substance to adsorb on an involatile substance; (ii) covering an aromatic substance with a membrane made of an impermeable material; (iii) preventing the diffusion of an aromatic substance by lowering the moisture content of a product; or (iv) allowing an aromatic substance to form an inclusion complex with other substances, reported by Sugisawa, H., "Kagaku to Seibutsu", Vol.10, No.2, p.92, 1972).

The use of various sacchaides for retaining aroma has been proposed. For example, Japanese Patent Kokoku No. 37,062/77 discloses a method of admixing an aromatic substance with an oligosaccharide to form a solid material; Japanese Patent Kokoku No. 52,177/93 discloses the method of admixing an oily aromatic substance with anhydrous crystalline α-maltose, which was proposed by the same applicant as the present invention; Japanese Patent Kokoku No. 26,345/96 discloses a mothod of admixing an oily aromatic substance with anhydrous crystalline α-maltose and cyclodextrin to prepare a solid mixture, which was proposed by the same applicant; Japanese Patent Kokai No. 35,251/79 and Kokoku No. 986/93 disclose a method of allowing an aromatic substance to forming an inclusion complex with cyclodextrin. Due to the saccharides used, these methods, however, have some disadvantages of lesser adsorption level of aromatic substances, insufficient capability of retaining aromatic substances, restricted applicability to specific oily aromatic substances, and unsatisfactory solubility of the resulting mixture in their secondary proceedings.

α,α-Trehalose, whose industrial production has been developed in recent years, is useful for retaining aroma. For example, Japanese Patent Kokai No. 111,284/97 discloses a method for producing emulsified perfumeries, comprising the steps of admixing an aromatic substance with α,α-trehalose in the presence of water and an emulsifier and powdery aroma-retaining materials made from the emulsified perfumeries, and drying the mixture. Although these trehalose products have a relatively high aroma retaining property and stability, they have disadvantages on the adsorbed amount of aroma and the capability of retaining aroma due to their easy crystallization. Therefore, there still remains a necessity of finding more suitable saccharides.

Recently, a cyclic tetrasaccharide represented by Chemical Formula 1 (hereinafter, it is simply abbreviated as "CTS") was reported by Bradburk G.M., et al., *Carbohydrate Research,* Vol. 329, pp. 655-665, (2000). CTS is a non-reducing cyclic saccharide composed of four glucose molecules linked *via* the alternating α-1,3 and α-1,6 glycosidic bonds. The process disclosed therein was not suitable as an industrial scale production, and it was not revealed on its use and physical property in detail. As disclosed in International Publication Nos. WO 90,338/01 (PCT/JP01/04276) and WO 10,361/02 (PCT/JP01/06412), the present inventors have established the process for producing CTS from starch and phytoglycogen with a low cost.

Chemical Formula 1:

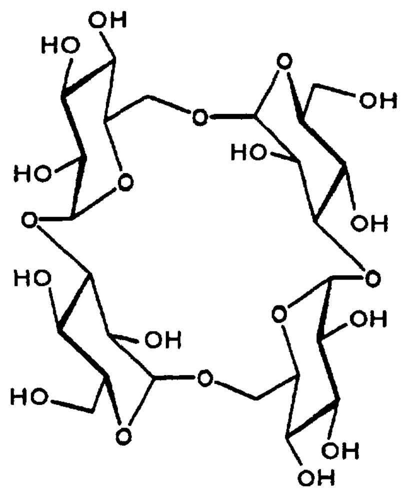

DISCLOSURE OF INVENTION

The object of the present invention is to solve the above problems of conventional methods for preparing aroma-retaining materials. Concretely, usual aromatic substances tend to denature, deteriorate and evaporate even when stored for a relatively short period of time due to lesser adsorption level of aromatic substance, insufficient capability of retaining aroma, and insufficient stability of aroma-retaining materials. The first object of the present invention is to provide a method for retaining aroma useful for various fields without fear of affecting aroma and aroma-retaining materials for the purpose of increasing the retaining amount of aroma, the long term reservation of aroma contained in aroma-retaining materials, and imparting stability to aroma-retaining materials. The second object of the present invention is to provide an aroma-retaining material having a satisfactory stability and capability of retaining aroma for a relatively long period of time. The third object of the present invention is to provide a composition comprising an aroma-retaining material. The fourth object of the present invention is to provide an aroma-retaining agent capable of retaining aroma for a relatively long period of time. The fifth object of the present invention is to provide a bacteriostat and/or bactericide comprising the agent having a sustained-release property.

The present inventors have eagerly studied the use of some kinds of saccharides, particularly CTS (industrially produced form starch or phytoglycogen material) or a mixture of CTS and its saccharide derivative(s) to attain the above objects. As a result, they found that CTS or a mixture of CTS and its saccharide derivative(s) was useful for retaining aroma of an aromatic substance. They accomplished the present invention by establishing a novel method for retaining aroma, a novel aroma-retaining material, a novel composition comprising an aroma-retaining material, a novel agent containing CTS or a mixture of CTS and its saccharide derivative(s), and a novel bacteriostat and/or bactericide comprising the agent having a sustained-release property.

BEST MODE FOR CARRYING OUT THE INVENTION

Aromatic substance as referred to as in the present invention is not restricted to a specific one, for example; extracts from citrus such as an orange, lemon, lime, and grapefruit; vegetable oils such as an essential flower oil, peppermint oil, spearmint oil, spice oil, and herb oil; plant extracts such as a kola nut extract, coffee extract, vanilla extract, cocoa extract, black tea extract, spice extract, and herb extract; essential oils extracted from an animal tissue; natural aroma materials including a tissue containing thereof; derivatives of natural aroma materials, chemically or semichemically synthesized aromatic substances, and mixtures thereof; can be used in the present invention. Further, compositions comprising aromatic substances for seasoning other compositions or fermented foods can be used in the present invention, for example; alcohols such as "*sake*" (Japanese rice wine), whiskey, brandy, liqueur, and *mirin* (Japanese sweet rice wine); dried bonito flake; boiled-dried fish; dried squid; oarweed; shrimp; *shiitake* mushroom; or extracts thereof; extracts such as bouillon, fishery extracts, vegetable extracts, and fruit extracts, or seasonings from the extracts; fermented foods such as soy sauce, fish sauce, *miso* (soybean paste), *mirin*, vinegar, and alcohols.

CTS or a mixture of CTS and its saccharide derivative(s) usable for admixing with aromatic substances or

compositions comprising aromatic substances is not specifically limited on their origin and preparation. For example, they can be produced in a usual fermentation, enzyme, or organic-chemistry technique. The reaction mixtures prepared by the above methods can be used intact as CTS or a mixture of CTS and its saccharide derivative(s). Optionally, it can be partially or perfectly purified before use. CTS or a mixture of CTS and its saccharide derivative(s) can be enzymatically produced from amylaceous materials or saccharide derivatives thereof with as following; (i) converting panose into CTS by an α-isomaltosyl transferring enzyme, which was disclosed by the present inventors in International Publication No. WO 90,338/01; (ii) directly producing CTS or a mixture of CTS and its saccharide derivative(s) from amylaceous substances using α-isomaltosyl glucosaccharide-forming enzyme and α-isomaltosyl transferring enzyme in combination, which was disclosed by the same applicant as the present invention in International Publication No. WO 10,361/02. Since these enzymatic methods produce CTS or its saccharide derivative(s) from abundant and inexpensive amylaceous substances efficiently and at a low cost, they are useful for industrial production of them. CTS has been known to be in various forms such as amorphous anhydrate, crystalline anhydrate, crystalline monohydrate and crystalline pentahydrate. CTS used in the present invention can be any of such forms. Particularly, when an aroma-retaining material in the form of a powder or solid is produced by mixing CTS and an aromatic substance containing moisture, CTS in the form of a crystalline anhydrate, crystalline monohydrate or amorphous anhydrate can be advantageously used because they act as a dehydrating agent having an advantageous of dehydrating capability.

Materials comprising other saccharides alone with CTS and its saccharide derivative(s) can be also used in the present invention. "Saccharide derivatives of CTS" as referred to as in the present invention means derivatives of CTS having one or more glycosyl groups which are same or different each other. A mixture of CTS and its saccharide derivative(s) is usually in the form of a saccharide solution containing CTS, its saccharide derivative(s) (having one or more glucoses positioning at one or more hydroxly groups in CTS), and/or other saccharides such as glucose, maltooligosaccharides, and maltodextrins obtainable by allowing α-isomaltosyl glucosaccharide-forming enzyme and α-isomaltosyl-transfer enzyme in combination to act on amylaceous substances. Further, it can be purified using ion-exchange resins partially or perfectly. According to Japanese Patent Application No. 67,282/01, disclosed by the same inventors as the present invention, saccharide derivatives which are formed by transferring one or more glucose residues (e.g. α-D-glucopyranosyl, β-D-galactopyranosyl and β-D-chitosaminyl) to one or more hydroxyl groups of CTS or its saccharide derivative(s), and which are produced by allowing one or more saccharide transferring enzymes (e.g. cyclomaltodextrin glucanotransferase, β-galactosydase, α-galactosydase, and lysozyme) to act on the mixture in the presence of saccharide substrates (e.g. monosaccharide, oligosaccharide, and polysaccharide) can be also used. Furthermore, the above saccharide derivatives can be purified partially or perfectly.

The aroma-retaining material of the present invention can be prepared by admixing (i) an aromatic substance (s) or a composition thereof, (ii) CTS or a mixture of CTS and its saccharide derivative(s) in the form of a syrup, mascuite, solid, or powder, (iii) optional water, and (iv) optional other ingredients. When the resulting aroma-retaining material is in the form of a liquid or semi-solid, it can be converted into the form of a solid or powder by conventional drying such as heating, drying *in vacuo*, spray-drying, or lyophilizing. Particularly, the aroma-retaining material in a powder form can be advantageously produced by the steps of homogeneously admixing aromatic substances and saccharides with water and/or emulsifier, and drying the resulting mixture.

CTS or a mixture of CTS and its saccharide derivative(s) used for mixing with an aromatic substance can be freely chosen from those with various forms such as syrup, mascuite, solid, powder, and mixture thereof, considering the property of the aromatic substance or other ingredients, stability of the aromatic substance, forms of aroma-retaining materials, and workability. The term "solid or powder containing CTS or a mixture of CTS and its saccharide derivative (s)" as referred to as in the present invention means a saccharide mixture in the form of a solid or powder, which contains (i) CTS amorphous anhydrate, CTS crystalline anhydrate, CTS crystalline hydrate, CTS crystalline anhydrate, or a mixture thereof, (ii) one or more saccharide derivatives of CTS in the form or a solid or powder, and (iii) other saccharides in the form of a solid or powder.

The composition comprising aromatic substances with CTS or a mixture of CTS and its saccharide derivative (s) is useful as an aroma-retaining agent. Further, it can be used in an appropriate amount of one or more monosaccharides such as xylose, arabinose, glucose, fructose, psicose, and mannose, oligosaccharides such as maltose, isomaltose, sucrose, lactose, α,α-trehalose, neotrehalose, panose, maltotriose, maltotetraose, maltopentaose, α-cyclodextrin, β-cyclodextrin, γ-cyclodextrin, and derivatives thereof, polysaccharide such as high-molecular dextrin, guar gum, arabic gum, pullulan, and hydroxyethyl starch. Furthermore, intense sweeteners such as sucralose, acesulfame-K, stevia, glycotransferring stevia, glycyrrhizin, saccharine, and L-aspartyl-L-phenylalanine methylester can be freely used.

Aroma-retaining materials having retaining property and stability can be obtained by mixing the solid and/or powder containing CTS or a mixture of CTS and its saccharide derivative(s) with an aromatic substance or a composition containing aromatic substances, optionally dissolving the mixture in an organic solvent, and, optionally, adding other ingredients to the resulting mixture. By co-existing these saccharides and aromatic substance containing organic solvent in an amount sufficient to dissolve a part of the saccharides, and optionally, other ingredients, solid products can

be obtained. Further, the resulting solid products can be freely processed into powdery aroma-retaining products.

The term "allowing CTS or a mixture of CTS and its saccharide derivative(s), aroma-retaining materials, and bacteriostat or bacteriocide to incorporate into" as referred to as in the present invention means contacting the above ingredients with other ingredients according to usual methods of mixing, kneading, dissolving, melting, dispersing, suspending, emulsifying, soaking, permeating, dispersing, applying, coating, spraying, injecting, crystallizing, and solidifying. The term "allowing to co-exist with aroma-retaining materials in the form of a bacteriostat or bacteriocide" as referred to as in the present invention means separately co-existing an aroma-retaining material(s) and a product as in the case of using the function of a vaporizing aromatic substance, for example, co-existing the aroma-retaining materials placed in a sealed or semi-sealed container.

Proportion of CTS or a mixture of CTS and its saccharide derivative(s) to an aromatic substance is not specifically restricted as long as the aromatic substance is satisfactorily adsorbed and retained, usually, in an amount of 1 to 10,000, desirably, 10 to 5,000 parts by weight to one part by weight of an aromatic substance(s) on a dry solid basis. When the amount of CTS or the mixture is lower than the lower limit, the property of retaining aroma will be more insufficient although the amount of the adsorbed aroma substance by weight is larger. While, when the amount of CTS or the mixture is higher than the upper limit, the workability during preparation and the physical properties of the composition will become worse.

The aroma-retaining material of the present invention is suitable as an agent for retaining aroma and/or sustained-releasing agent containing aromatic substances because it inhibits or prevents aromatic substances to evaporate and disappear, and prolongs the time until completion of its evaporating and disappearing, and retaining the aroma for a relative long period of time. Examples of such are alcohols, seasonings including soup stocks and fermented foods can be preserved for a relatively long period of time while retaining their inherent flavors of aromatic substances and keeping their original quality. Aromatic substances having an additional effect can be used as a preparation gradually performing the effect. Compositions, having the effect as a bacteriostat and/or bactericide or repellent, such as alcohols including ethanol, organic acids including acetic acid, hinokithiol, and wasabiol, can be advantageously used for products susceptive to the affect of microorganic contaminants or harmful insects by mixing or co-existing the aroma-retaining materials in foods, cosmetics, pharmaceuticals, clothing, or commodities because they gradually released aromatic substances and exert effect of bacterostat and/or bacterocide or repellent. Compositions having some effects on mind and body and/or the prevention and treatment of diseases, such as essential oils and extracts form herbs including lavender, rosemary, and chamomile, can be freely used as a deodorizer gradually releasing aromatic substances or a source of aromatic substances for aroma therapy such as a bath or herb tea by dissolving into a hot water.

The aroma-retaining material obtainable by the above-mentioned methods can be used as the following food compositions; beverages; powdered beverages; seasonings; sweeteners; Japanese confectioneries; cakes; water ices; syrups; pastes; pickles; processed marine products; processed livestock food products; prepared foods; dairy products; retort pouches; health foods; feeding stuffs and pet foods. Particularly, the aroma-retaining material of the present invention is suitable as a raw material for health foods because it retains aroma for a relatively long period of time and exerts the effect for intestinal disorders and decreases cholesterol due to the effect as a dietary fiber by the contained CTS or the mixture of CTS and its saccharide derivative(s). Furthermore, the aroma-retaining material can be used as cosmetics such as a soap, shampoo, rinse, body lotion, tooth paste, lip cream, agent for hair restoration, hairdressing, and bath salt, pharmaceuticals such as pack, buccal, cataplasm, oral agent, agent for percutaneous absorption, agent for permucotaneous absorption, powdery agents, and tablet, commodities such as agent for bed bath, detergent, fabric softener, fabric conditioner, aromatic, deodorant, reodorant, perfume, bacteriocide, fungicide, and repellent.

Varying depending on the kind and the form of a final product such as a food, beverage, pharmaceutical, and commodity, usually it contains 0.001 to 50 parts by weight of the aroma-retaining material to the raw materials or final products.

Following examples explain the present invention in more detail, but the present invention must not be restricted by the examples.

First, examples of producing enzymes for preparing CTS or a mixture of CTS and its saccharide derivative (s) are explained as follows:

Example A-1

Production of enzymes for producing CTS and its saccharide derivatives

α-Isomaltosylglucosaccharide-forming enzyme and α-isomaltosyl-transferring enzyme required for producing CTS from starch material were prepared at first. A liquid culture medium, consisting of 4.0%(w/v) of "PIN-DEX #4", a partial starch hydrolyzate commercialized by Matsutani Chemical Industries., Co. Ltd., Tokyo, Japan, 1.8%(w/v) of "ASAHIMEAST", a yeast extract commercialized by Asahi Breweries, Ltd., Tokyo, Japan, 0.1%(w/v) of dipotassium

phosphate, 0.06%(w/v) of sodium phosphate dodecahydrate, 0.05%(w/v) magnesium sulfate heptahydrate, and water, was placed in 500-ml Erlenmeyer flasks in a volume of 100 ml each, sterilized by autoclaving at 121°C for 20 min, cooled, and then seeded with *Bacillus globisporus* C9 strain, FERM BP-7143, followed by culturing under rotary-shaking conditions at 27°C and 230 rpm for 48 hours for seed culture. About 20 L of a fresh preparation of the same liquid culture medium as used in the above seed culture were placed in a 30-L fermentor, sterilized by heating, and then cooled to 27°C and inoculated with 1%(v/v) of the seed culture, followed by culturing at 27°C and pH 6.0-8.0 for 48 hours under aeration-agitation conditions. After completion of the culture, the resulting culture, which had about 0.45 unit/ml of α-isomaltosylglucosaccharide-forming enzyme, about 1.5 units/ml of α-isomaltosyl-transferring enzyme, and about 0.95 unit/ml of cyclic tetrasaccharide-forming activity, was centrifuged at 10,000 rpm for 30 min to obtain about 18 L of a supernatant. When measured for enzymatic activity, the supernatant had about 0.45 unit/ml of the α-isomaltosylglucosaccharide-forming enzyme, i.e., a total enzymatic activity of about 8,110 units; about 1.5 units/ml of α-isomaltosyl-transferring enzyme, i.e., a total enzymatic activity of about 26,900 units.

The activities of these enzymes were measured as follows: The activity of α-isomaltosylglucosaccharide-forming enzyme was measured by the steps of dissolving maltotriose in 100 mM acetate buffer (pH6.0) to give a concentration of 2% (w/v) for a substrate solution, adding a 0.5 ml of an enzyme solution to a 0.5 ml of the substrate solution, enzymatically reacting the mixture solution at 35°C for 60 min, suspending the reaction mixture by boiling for 10 min, and quantifying maltose, among the isomaltosyl maltose and maltose formed in the reaction mixture, on conventional HPLC. One unit activity of the α-isomaltosylglucosaccharide-forming enzyme is defined as the amount of the enzyme that forms 1μmole of maltose per minute under the above conditions. Throughout the specification, the activity of the α-isomaltosylglucosaccharide-forming enzyme means the unit(s) measured as above.

The activity of α-isomaltosyl-transferring enzyme was measured by the steps of dissolving panose in 100 mM acetate buffer (pH6.0) to give a concentration of 2% (w/v) for a substrate solution, adding a 0.5 ml of an enzyme solution to 0.5 ml of the substrate solution, enzymatically reacting the mixture by boiling for 10 min, and quantifying glucose, among the CTS and glucose formed in the reaction mixture, by the glucose oxidase method. One unit activity of the α-isomaltosyl-transferring enzyme is defined as the enzyme amount that forms 1μmole of glucose per minute under the above enzymatic reaction conditions. Throughout the specification, the enzymatic activity of the α-isomaltosyl-transferring enzyme means the unit(s) measured as above.

The cyclic tetrasaccharide-forming activity is measured by the steps of dissolving "PINE-DEX #100", a partial starch hydrolysate commercialized by Matsutani Chemical Industries., Co., Ltd., Tokyo, Japan, in 50 mM acetate buffer (pH 6.0) to give a concentration of 2%(w/v) for a substrate solution, adding 0.5 ml of an enzyme solution to 0.5 ml of the substrate solution, enzymatically reacting the mixture solution at 35°C for 60 min, suspending the reaction mixture by boiling for 10 min, and then further adding to the resulting mixture one milliliter of 50 mM acetate buffer (pH 5.0) with 70 units/ml of "TRANSGLUCOSIDASE L AMANO™", an α-glucosidase commercialized by Amano Pharmaceutical Co., Ltd., Aichi, Japan, and 27 units/ml of glucoamyrase, commercialized by Nagase Biochemicals, Ltd., Kyoto, Japan, and incubated at 50°C for 60min, inactivating the retaining enzymes by heating at 100°C for 10min, and quantifying cyclotetrasaccharide on HPLC similarly as in Experiment 1. One unit of cyclotetrasaccharide-forming activity is defined as the enzyme amount that forms one micromole of cyclotetrasaccharid per minute under the above enzymatic reaction conditions. Throughout the specification, the cyclic tetrasaccharide-forming activity means the activity (units) measured as above.

Example A-2

Preparation of enzymes derived form *Bacillus globisporus* C9

About 18 L of the supernatant in Example A-1 was salted out with 80% saturated ammonium sulfate and allowed to stand at 4°C for 24 hours, and the formed sediments were collected by centrifugation at 10,000 rpm for 30 min, dissolved in 10 mM phospate buffer (pH7.5), and dialyzed against a fresh preparation of the same buffer to obtain about 400 ml of a crude enzyme solution with 8,110 units of the α-isomaltosylglucosaccharide-forming enzyme, 24,700 units of α-isomaltosyl-transferring enzyme, and about 15,600 units of cyclic tetrasaccharide-forming activity. The crude enzyme solution was subjected to ion-exchange chromatography using 1,000 ml of "SEPABEADS FP-DA13" gel, an ion-exchange resin commercialized by Mitsubishi Chemical Industries, Ltd., Tokyo, Japan. The α-isomaltosylglucosaccharide-forming enzyme and α-isomaltosyl transferring enzyme were eluted as non-adsorbed fractions without adsorbing on the ion-exchange resin. The resulting enzyme solution was dialyzed against 10 mM phosphate buffer (ph7.0) with 1 M ammonium sulfate, and the dialyzed solution was free from impurities by centrifuging, and subjected to affinity chromatography using 500 ml of "SEPHACRYL HR S-200", a gel commercialized by Amersham Corp., Div. Amersham International, Arlington Heights, IL, USA. Enzymatically active components were adsorbed on the gel, and when sequentially eluted with a linear gradient decreasing from 1 M to 0 M of ammonium sulfate and a linear gradient increasing from 0 mM to 100 mM of maltotetraose, the α-isomaltosylglucosaccharide-forming enzyme and the α-isomaltosyl-transferring enzyme were separatory eluted, i.e., the former was eluted with the linear gradient of maltotetraose at about 30 mM and the latter was eluted with the linear gradient of ammonium sulfate at about 0 M. Thus, fractions with α-isomaltosyl-transferring activity and those with the α-isomaltosylglucosaccharide-forming activity.

Methods for separatory purifying the α-isomaltosylglucosaccharide-forming enzyme and α-isomaltosyl-transferring enzyme are described in the below:

Example A-3

Purification of α-isomaltosylglucosaccharide-forming enzyme derived from *Bacillus globisporus* C9

A fraction of the α-isomaltosylglucosaccharide-forming enzyme, obtained example A-2, was dialyzed against 10 mM phosphate buffer (pH 7.0) containing 1 M ammonium sulfate. The dialyzed solution was free from insoluble impurities by centrifuging and fed to hydrophobic chromatography using 350 ml of "BUTYL-TOYOPEARL 650M", a gel commercialized by Tosoh Corporation, Tokyo, Japan. The enzyme was adsorbed on the gel and eluted at about 0.3 M ammonium sulfate when eluted with a linear gradient decreasing from 1 M to 0 M of ammonium sulfate, followed by collecting fractions with the enzyme activity. The fractions were pooled and again dialyzed against 10 mM phosphate buffer (pH 7.0) containing 1 M ammonium sulfate. The resulting dialyzed solution was centrifuged, and the resulting supernatant free from insoluble impurities was fed to affinity chromatography using "SEPHACRYL HR S-200" gel to purify the enzyme.

Example A-4

Purification of α-isomaltosyl-transferring enzyme derived form *Bacillus globisporus* C9

The fraction of the α-isomaltosyl-transferring enzyme, separated from the α-isomaltosylglucosaccharide-forming enzyme using the affinity chromatography described in Example A-2, was dialyzed against 10 mM phosphate buffer (pH 7.0) containing 1 M ammonium sulfate. The dialyzed solution was centrifuged, and the resulting supernatant free from insoluble impurities was fed to hydrophobic chromatography using 350 ml of "BUTYL-TOYOPEARL 650M", a gel commercialized by Tosoh Corporation, Tokyo, Japan. The enzyme was adsorbed on the gel and eluted at about 0.3 M ammonium sulfate when eluted with a linear gradient decreasing from 1 M to 0 M of ammonium sulfate, followed by collecting fractions with the enzyme activity. The fractions were pooled and again dialyzed against 10 mM phosphate buffer (pH 7.0) containing 1 M ammonium sulfate. The resulting dialyzed solution was centrifuged, and the resulting supernatant free from insoluble impurities was fed to affinity chromatography using "SEPHACRYL HR S-200" gel to purify the enzyme.

Following explains the method for preparing the mixture of CTS and its saccharide derivative(s).

Example B-1

Preparation of a syrup containing a mixture of CTS and its saccharide derivative(s)

A potato starch was prepared into an about 6% starch suspension, admixed with calcium chloride to give a final concentration of 0.1 %, adjusted to pH 6.0, further admixed with an α-amylase. To the resulting liquefied solution was added 2 units of α-isomaltosylglucosaccharide-forming enzyme prepared in Example A-3 and 6 units of α-isomaltosyl-transferring enzyme prepared in Example A-4 per gram starch on dry solid basis followed by the enzymatic reaction for 48 hours. The reaction mixture was heated to and kept at 95°C for 10 min to be inactivated, and then, it was decolored, desalted, filtered, and concentrated by conventional methods. As a result, an 80% saccharide syrup containing 0.6% glucose, 1.5% isomaltose, 12.3% maltose, 63.5% CTS, and 5.2% saccharide derivatives of CTS combining with one or more glucoses was prepared.

Example B-2

Preparation of CTS

About 100 L of a 4% (w/v) aqueous solution of corn phytoglycogen, commercialized by Q.P. Corporation, Tokyo, Japan, was prepared, adjusted to pH 6.0 and 30°C, and then admixed with 1 unit/g solid of a purified specimen of α-isomaltosylglucosaccharide-forming enzyme obtained in Example A-3 and 10 units/g solid of a purified specimen of α-isomaltosyl-transferring enzyme obtained in Example A-4, followed by the incubation for 48 hours. After completion of the reaction, the reaction mixture was heated at 100°C for 10 min with the aim of inactivating the retaining enzymes.

The resulting reaction mixture was adjusted to pH 5.0 and 45°C, and then treated with α-glucosidase and glucoamylase similarly as in Example A-1 to hydrolyze the retaining reducing oligosaccharides, etc. The resulting mixture was adjusted to pH 5.8 by the addition of sodium hydroxide and then incubated at 90°C for one hour with the aim of inactivating the retaining enzymes and filtered with the aim of removing insoluble substances. The filtrate was concentrated using a reverse osmosis membrane to give a concentration of about 16% on a dry solid basis, and the concentrate was in a usual manner decolored, desalted, filtered, and concentrated. As a result, about 6.2kg of a saccharide solution with a solid content of about 3,700g was obtained.

The saccharide solution as fed to a column packed with about 225 L of "AMBERLITE CR-1310 (Na-form)", an ion-exchange resin commercialized by Japan Organo Co., Ltd., Tokyo, Japan, and chromatographed at a column temperature of 60°C and a flow rate of about 45 L/h. While the saccharide composition of eluate from the column was monitoring by HPLC as described in Example A-1, fractions of CTS with a purity of at least 98% were collected.

Example B-3

Preparation of CTS crystalline hydrate

A fraction of CTS with a purity of at least 98%, obtained by the above method, was concentrated by evaporation to give a concentration of about 50% on a dry solid basis. About 5kg of the concentrate was placed in a cylindrical plastic vessel and then crystallized to obtain a white crystalline powder by lowering the temperature of the concentrate from 65°C to 20 °C over about 20 hours under gentle rotatory conditions. The above crystallized concentrate was separated by passing through a centrifugal filter to obtain 1,360 g of a crystalline product by wet weight, which as then further dried at 60°C for three hours to obtain 1,170 g of a crystalline powder of CTS. HPLC measurement of the crystalline powder revealed that it contained CTS with a quite high purity of at least 99.9%. When analyzed on powder X-ray diffraction analysis, the CTS in a crystalline powder form had a diffraction spectrum having characteristic main diffraction angles (2θ) of 10.1°, 15.2°, 20.3°, and 25.5°. The Karl Fischer method of the crystalline powder revealed that it had a moisture content of 13.0%, resulting in a finding that it as a crystal of CTS having five moles of water per one mole of the crystal.

Example B-4

Preparation of CTS crystalline monohydrate

CTS crystalline pentahydrate in the form of a powder, obtained according to the method in Example B-3, was placed in a glass vessel, and kept in an oil bath, which had been preheated at 140°C, for 30 min. The powder X-ray diffraction analysis of the CTS powder thus obtained gave a characteristic diffraction spectrum having main diffraction angles (2θ) of 8.3°, 16.6°, 17.0°, and 18.2°. The Karl Fischer method of the crystalline powder revealed that it had a moisture content of about 2.7%, resulting in a finding that it was a crystal of CTS having one mole of water per one mole of the crystal.

Example B-5

Preparation of CTS crystalline anhydrate

CTS crystalline pentahydrate in the form of a powder, obtained by the method in Example B-3, was dried *in vacuo* at 120°C for 16 hours. The powder X-ray diffraction analysis of the above CTS gave characteristic diffraction spectra having main diffraction angles (2θ) of 10.8°, 14.7°, 15.0°, 15.7°, and 21.5°. The Karl Fischer method of the resulting crystalline powder revealed that it had a moisture content of about 0.2%, meaning that it was substantially anhydrous.

Example B-6

Preparation of CTS amorphous anhydrate

Fractions containing CTS with a purity of at least 98%, obtained by the method in Example B-2, were in a usual manner desalted, decolored, and filtered to obtain a concentrate having a solid concentration of 50%. The concentrate thus obtained was promptly freezed at -80°C, lyophilized, and further dried *in vacuo* at 80°C for three hours. The resulting dried product was pulverized with a pulverizer. The powder X-ray diffraction analysis of the resulting powder revealed that the powder was amorphous since it gave no characteristic diffraction spectrum. The Karl Fischer

method of the powder revealed that it had a moisture content of about 0.3%, meaning that it was substantially anhydrous.

Example C-1

Preparation of aroma-retaining material using CTS crystalline pentahydrate, CTS crystalline monohydrate, CTS crystalline anhydrate, or CTS amorphous anhydrate

CTS crystalline pentahydrate, prepared by the method described in Example B-3, CTS crystalline monohydrate, prepared by the method described in Example B-4, CTS crystalline anhydrate, prepared by the method described in Example B-5, or CTS amorphous anhydrate prepared by the method described in Example B-6, was used for preparation of aroma-retaining material. "ISOELEAT P™", a branched cyclodextrin commercialized by Maruha corporation, Tokyo, Japan, anhydrous trehalose prepared by the method described in Japanese Patent Kokai No. 111,284/97, "PINEFIBRE™", a dextrin commercialized by Matsutani Chemical Industries Co., Ltd., Tokyo, Japan, or soluble starch in a reagent grade commercialized by Katayama Chemical Industries, Co., Ltd., Tokyo, Japan, was dried *in vacuo* at 80°C for 16 hours as control saccharides. Ethanol or acetic acid in a liquid form as an aromatic substance was gradually admixed with 10g of the above saccharide, and the mixture was placed into a glass triturator and stirred with a glass rod until the resulting mixture did not keep a powder form. The resulting mixture was weighed. The weight of ethanol or acetic acid was calculated by subtracting the pre-measured saccharide weight. The weight of ethanol or acetic acid adsorbed on 1g of each saccharide was in Table 1.

Table 1

| Saccharide | Amount of Adsorbed Ethanol/Acetic Acid(g) | |
|---|---|---|
| | Ethanol | Acetic acid |
| CTS crystalline pentahydrate | 0.32 | 0.27 |
| CTS crystalline monohydrate | 0.45 | 0.35 |
| CTS crystalline anhydrate | 0.50 | 0.47 |
| CTS amorphous anhydrate | 0.49 | 0.47 |
| Branched cyclodextrin | 0.30 | 0.23 |
| Anhydrous trehalose | 0.25 | 0.18 |
| Dextrin | 0.21 | 0.26 |
| Soluble starch | 0.12 | 0.06 |

From the result in Table 1, CTS crystalline pentahydrate, CTS crystalline monohydrate, CTS crystalline anhydrate or CTS amorphous anhydrate has stronger effect on adsorbing ethanol or acetic acid in a liquid form than the control saccharides. Particularly, it is revealed that CTS crystalline anhydrate and CTS amorphous anhydrate have the strongest effect.

Example C-2

Effect of suppressing volatilization

About 5g of an ethanol-retaining material with CTS crystalline anhydrate, branched cyclodextrin, anhydrous trehalose, or dextrin, prepared freshly by the method described in Example C-1, was taken into a 20 ml volume of glass vials. The vials were placed under normal pressure and room temperature for the purpose of evaporating ethanol. The amount of retained ethanol in samples freshly prepared, or after two hours later, four hours later, eight hours later, or 24 hours later, was measured by dissolving each sample in about 10 ml of water, diluting it with water to give a volume of 100ml in a glass measuring flask, and measuring the amount of ethanol dissolved in the resulting aqueous solution by a gas chromatography. In the case of a soluble starch as a sample, additional centrifuging was carried out after diluting up to 100ml. The above gas chromatography was carried out using "GC-14B" (a gas liquid chromatography commercialized by Shimazu Corporation, Kyoto, Japan) equipped with "TC-5" (a capillary column sized 0.53mm in diameter and 15m in length, commercialized by GL Sciences Inc., Tokyo, Japan). The amount of retained ethanol per 1g of each material is in Table 2.

Table 2

| Saccharide | Amount of Retained Ethanol (mg) | | | | |
|---|---|---|---|---|---|
| | Freshly-Prepared | 2h Later | 4h Later | 8h Later | 24h Later |
| CTS crystalline anhydrate | 333 (100%) | 335 (101%) | 290 (87%) | 10 (3%) | 5 (2%) |
| Branched cyclodextrin | 230 (100%) | 110 (48%) | 30 (13%) | 7 (3%) | 3 (1%) |
| Anhydrous trehalose | 203 (100%) | 103 (51%) | 17 (8%) | 8 (4%) | 5 (2%) |
| Dextrin | 176 (100%) | 27 (15%) | 9 (5%) | 6 (3%) | 4 (2%) |
| Soluble starch | 104 (100%) | 7 (7%) | 4 (4%) | 2 (2%) | 1 (1%) |
| In this table, each value in the parentheses means a relative percentage to the ethanol amount (100%) just after its preparation. | | | | | |

As evident from the result in Table 2, CTS crystalline anhydrate retains the adsorbed ethanol more remarkably and evaporates the adsorbed ethanol more gradually compared to the branched cyclodextrin, anhydrous trehalose, dextrin, and soluble starch. Therefore, it has only the effect of adsorbing aromatic substance but also satisfactory retains aromatic substance. Additionally, 24 hours later, it lost adsorbed ethanol due to evaporation similarly as in the other saccharides. The result shows that the aroma-retaining material of the present invention is useful for a sustained-releasing agent because it gradually releases the aromatic substance.

Example C-3

Preparation of aroma-retaining materials containing CTS crystalline anhydrate

Fifty-five kinds of aroma-retaining materials containing CTS crystalline anhydrate prepared by the method described in Example B-5, and one selected from the group consisting of methanol, ethanol, 1-propanol, 1-butanol, 1-pentanol, 1-octanol, 2-propanol, 2-methyl-1-propanol, 2-butanol, 2-methyl-2-propanol, 3-methyl-1-propanol, benzyl alcohol, phenethyl alcohol, 2-aminoethanol, diacetone alcohol, 1,4-butanediol, 1-hexanol, O-methoxyphenol, 3-phenyl-1-propanol, benzene, toluene, benzyl chloride, chloroform, ethyl acetate, diethyl ether, petroleum ether, α-terpinene, α-pinene, β-pinene, α-terpineol, terpinen-4-ol, limonene, geraniol, linalool, citronellal, citronellol, γ-dodecanolactone, pentylacetate, bornylacetate, allylisothiocyanate, t-butylisothiocyanate, hinokithiol, lemon oil, rosemary oil, lavender oil, formic acid, acetic acid, propionic acid, butyric acid, valeric acid, capronic acid, octanic acid, isobutyric acid, and isovaleric acid were prepared. The following explains the experimental method in detail. A small amount of each of the above aromatic substances was added to 4g of CTS in a glass triturator and mixed repeatedly to be adsorbed on CTS until the mixture did not keep in a powder form. The amount of the adsorbed aromatic substances was calculated by measuring the weight of the resulting powders and subtracting their starting powder weight (4g). The amount of the adsorbed aromatic substances per 1g of CTS crystalline anhydrate was shown in Table 3.

Table 3

| Aromatic substance | Amount of Adsorbed Substance (g) | Aromatic substance | Amount of Adsorbed Substance (g) |
|---|---|---|---|
| Methanol | 0.61 | α-Pinene | 0.35 |
| Ethanol | 0.50 | β-Pinene | 0.30 |
| 1-Propanol | 0.46 | α-Terpineol | 0.33 |
| 1-Butanol | 0.22 | Terpinen-4-ol | 0.34 |
| 1-Pentanol | 0.31 | Limonene | 0.42 |

Table 3 (continued)

| Aromatic substance | Amount of Adsorbed Substance (g) | Aromatic substance | Amount of Adsorbed Substance (g) |
|---|---|---|---|
| 1-Octanol | 0.28 | Geraniol | 0.32 |
| 2-Propanol | 0.48 | Linalool | 0.42 |
| 2-Methyl-1-Propanol | 0.36 | Citronellal | 0.46 |
| 2-Butanol | 0.29 | Citronellol | 0.39 |
| 2-Methyl-2-Propanol | 0.32 | γ-Dodecanolactone | 0.35 |
| 3-Methyl-1-Propanol | 0.36 | Pentylacetate | 0.46 |
| Benzyl Alcohol | 0.43 | Bornylacetate | 0.48 |
| Phenethyl Alcohol | 0.40 | Allylisothiocyanate | 0.37 |
| 2-Aminoethanol | 0.13 | t-Butylisothiocyanate | 0.36 |
| Diacetone Alcohol | 0.28 | Hinokithiol | 0.29 |
| 1,4-Butanediol | 0.28 | Lemon Oil | 0.28 |
| 1-Hexanol | 0.41 | Rosemary Oil | 0.31 |
| *o*-Methoxyphenol | 0.42 | Lavender Oil | 0.29 |
| 3-Phenyl-1-Propanol | 0.52 | Formic Acid | 0.24 |
| Benzene | 0.14 | Acetic Acid | 0.47 |
| Toluene | 0.29 | Propionic Acid | 0.50 |
| Benzyl Chloride | 0.32 | Butyric Acid | 0.55 |
| Chloroform | 0.32 | Valeric Acid | 0.47 |
| Etyl Acetate | 0.41 | Capronic Acid | 0.47 |
| Diethyl Ether | 0.17 | Octanic Acid | 0.47 |
| Petroleum Ether | 0.21 | Isobutyric Acid | 0.51 |
| α-Terpinene | 0.37 | Isovaleric Acid | 0.46 |

As evident from the result in Table 3, CTS crystalline anhydrate has an effect of adsorbing aromatic substance in a liquid form such as alcohols, esters, ethers, essential oils, and organic acids, revealing that it can prepare aroma-retaining materials from various aromatic substances in the liquid form.

Example C-4

Preparation of aroma-retaining materials using CTS crystalline hydrate, CTS crystalline anhydrate, or CTS amorphous anhydrate, and test of their properties

The following example is a test to confirm the effect of CTS crystalline hydrate, CTS crystalline anhydrate, or CTS amorphous anhydrate on retaining, as an aromatic substance, lavender oil, citronellol, or phenethyl alcohol. CTS crystalline pentahydrate prepared by the method described in Example B-3, CTS crystalline anhydrate in Example B-5, and CTS amorphous anhydrate in Example B-6 were used as the test saccharides "ISOELEAT P®", a branched cyclodextrin commercialized by Maruha Corporation, Tokyo, Japan, was used as a control.

In the case of lavender oil, five parts by weight of each of the above saccharides were taken into a glass dish sized 90 mm in diameter and 20 mm in depth, admixed with one part by weight of lavender oil by stirring. Lavender oil was measured by the method described as follows; admixing 2 ml of diethyl ether with 1g of each of the above samples freshly prepared or after preserved at 25°C for 10 days; measuring the extracted lavender oil by gas chromatography using "GC-14B" (a gas chromatography commercialized by Shimazu corporation, Kyoto, Japan) equipped with "TC-Wax" (a capillary column sized 0.53mm in diameter and 25m in length commercialized by GL Sciences Inc., Tokyo, Japan.

In the case of citronellol or phenethyl alcohol, five parts by weight of each of the above saccharides were taken into a glass dish sized 90mm in diameter and 20mm in depth, admixed with one part by weight of citronellol or phenethyl alcohol, and mixed by stirring. The adsorbed citronellol or phenethyl alcohol was measured by admixing 2ml of diethyl ether with 1g of each of the above sample freshly prepared or after preserved at 25°C for 10 days, and measuring the extracted lavender oil by gas chromatography using "GC-14B" (a gas chromatography device commercialized by Shimazu corporation, Kyoto, Japan) equipped with "DB-5" (a capillary column sized 0.25mm in diameter and 30m in length commercialized by GL Sciences Inc., Tokyo, Japan).

The amount of the retained aromatic substance in each sample was calculated using the following formula, and the results were shown in Table 4.

Formula:

Relative amount of the retained Substance (%)

= {(an amount of aromatic substance in a sample preserved for 10 days)/(an amount of aromatic substance in a freshly prepared sample)} × 100

Table 4

| Aromatic substance | Amount and Relative Amount | Saccharide | | | |
|---|---|---|---|---|---|
| | | Sample 1 | Sample 2 | Sample 3 | Sample 4 |
| Lavender Oil | Amount of the Adsorbed Substance (mg/g saccharide) | 150 | 190 | 185 | 145 |
| | Relative Amount of the Retained Substance (%) | 1 | 12 | 8 | 0 |
| Citronellol | Amount of the Adsorbed Substance (mg/g saccharide) | 155 | 195 | 190 | 150 |
| | Relative Amount of the Retained Substance (%) | 30 | 42 | 40 | 9 |
| Phenethyl Alcohol | Amount of the Adsorbed Substance (mg/g saccharide) | 140 | 190 | 180 | 130 |
| | Relative Amount of the Retained Substance (%) | 3 | 16 | 12 | 0 |
| Sample 1: CTS Crystalline Hydrate Sample 2: CTS Crystalline Anhydrate<br>Sample 3: CTS Amorphous Anhydrate Sample 4: Branched Cyclodextrin | | | | | |

As evident from the result in Table 4, the freshly-prepared samples retained 150 mg/g of lavender oil in the case of CTS crystalline hydrate, 190 mg/g for CTS crystalline anhydrate, 185 mg/g for CTS amorphous anhydrte, and 145 mg/g for branched cyclodextrin as a control. CTS crystalline anhydrate and CTS amorphous anhydrate had about 1.3-fold higher adsorbing capacity of branched cyclodextrin. The freshly-prepared samples contained 155 mg/g of citronellol for CTS crystalline hydrate, 195 mg/g for CTS crystalline anhydrate, 190 mg/g for CTS amorphous anhydrate, and 150 mg/g within branched cyclodextrin as a control. CTS crystalline anhydrate and CTS amorphous anhydrate had about 1.2-fold higher capability of branched cyclodextrin. The freshly-prepared samples was contained 140 mg/g of phenethyl alcohol within CTS crystalline hydrate, 190 mg/g within CTS crystalline anhydrate, 180 mg/g within CTS amorphous anhydrate, and 130 mg/g within branched cyclodextrin as a control. CTS crystalline anhydrate and CTS amorphous anhydrate had about 1.4 to 1.5-fold capability of branched cyclodextrin.

In view of the retaining aromatic substances, in the case of the samples of lavender oil and phenethyl alcohol, the rate of retaining aromatic substance is relatively low. However, in view of the rate of retained aromatic substance, since CTS in any form, particularly CTS crystalline anhydrate and CTS amorphous anhydrate, have a higher rate than that of the branched cyclodextrin as a control, revealing to have a satisfactory retaining effect. In the case of the samples of citronellol, the rate of retaining aromatic substance is relatively high. The rate of CTS in any form is higher than that of the branched cyclodextrin as a control by about 3.3-fold to 4.7-fold.

Considering the above results, CTS crystalline hydrate, CTS crystalline anhydrate, and CTS amorphous anhydrate have a high capability for adsorbing and retaining aromatic substances depending on the kind of aromatic substance. Therefore, they are useful as the agent for retaining aroma in the fields of foods, cosmetics, and pharmaceuticals.

Example C-5

Herb extract-retaining material

To the mixture of 150 parts by weight of water, 30 parts by weight of carrageenan, and 50 parts by weight of hydrolyzed starch were mixed with 20 parts by weight of any one of the group consisting of CTS in the form of a pentahydrous crystalline, sucrose (granulated sugar) and "TREHA®", an α,α-trehalose product commercialized by Hayashibara Shoji, Inc., Okayama, Japan, and then, the resulting solution was sterilized by heating to 100°C for 15 min and cooling down to 40°C. One part by weight of a herb extract was admixed with the solution and emulsified with "TK-homomixer", a mixer commercialized by Tokushu Kika Kogyo Co., Ltd., Osaka, Japan. By spray-drying the resulting emulsion in a spray-dryer set to 120°C at the entrance temperature and 80°C at the exit temperature, a herb-extract-retaining material was obtained. The amount of the retained aroma substance in the sample was evaluated by 15 panels in such a manner of judging the samples about the strength of aroma after preserved in an opened container at room temperature for two months. The result judged in terms of three criteria was shown in Table 5. The criterion "good" means strength of aroma when CTS was used as a saccharide. The criterion "slightly good" means that strength of aroma is weaker than that with CTS. The criterion "no good" means that aroma was almost lost.

Table 5

| Saccharide | Evaluation | | |
|---|---|---|---|
| | Good | Slightly Good | No Good |
| CTS | 15 | 0 | 0 |
| Sucrose | 0 | 9 | 6 |
| α,α-Trehalose | 1 | 13 | 1 |

As evidence from the result in Table 5, only one panel of 15 panels judged trehalose as the same as in CTS in view of the strength of aroma, however, other panels judged sucrose or α,α-trehalose was interior to CTS as judged "slightly good" or "no good". The aroma-retaining material containing CTS of the present invention can be advantageously used as an agent for aroma source in the fields of foods, cosmetics, and pharmaceuticals because it is a stable aroma-retaining product during preservation. It can be advantageously used for health supplements or raw materials thereof because CTS also functions as a dietary fiber.

Example C-6

Grapefruit aroma-retaining material

Five parts by weight of sucrose fatty acid ester HLB15 grade, 45 parts by weight of "TETRUP®", a syrup commercialized by Hayashibara Shoji, Inc., Okayama, Japan, and 40 parts by weight of CTS crystalline pentahydrate prepared by the method described in Example B-3 were admixed and dissolved in 100 parts by weight of water, and then the resulting solution was sterilized by heating at 85 to 90°C for 15 min and cooling down to about 40°C. The resulting solution was admixed with 10 parts weight of grapefruit oil and emulsified. The resulting emulsion was dried in such a manner of being quickly frozen in -80°C to lose its moisture, heated up to 50°C, and vacuum-dried for three hours. The dried resultant was pulverized with a pulverizer to obtain a grapefruit aroma-retaining powder. The product is useful as an agent for aroma source in the fields of foods, cosmetics, pharmaceuticals, and commodities because it stably retains grapefruit oil.

Example C-7

Menthol aroma-retaining material

Twenty parts by weight of CTS amorphous anhydrate prepared by the method described in Example B-6 and

10 parts by weight of "TREHA®", a high-purity hydrous crystalline α,α-trehalose commercialized by Hayashibara Shoji, Inc, Okayama, Japan, were admixed with 10 parts by weight of water, dissolved by heating and cooling down to 50°C. To the resulting solution was admixed one part by weight of menthol to obtain a menthol aroma-retaining material. The product is useful as an agent for aroma source or perfuming agent in the fields of foods, cosmetics, pharmaceuticals, and commodities because it stably retains menthol.

Example C-8

Coffee aroma-retaining material

Twenty parts by weight of CTS crystalline pentahydrate prepared by the method described in Example B-3 and 20 parts by weight of "SUMMALT®", a maltose commercialized by Hayashibara Shoji, Inc., Okayama, Japan, 10 parts by weight of "TETRUP®", a starch syrup commercialized by Hayashibara Shoji, Inc., Okayama, Japan, were admixed with 10 parts by weight of water, dissolved by heating, and cooled down to 50°C. To the resulting solution was added 10 parts by weight of a commercialized coffee powder to obtain a solution containing coffee. The solution was dried in such a manner of being quickly frozen in -80°C to lose its moisture, heated up to 60°C, and vacuum-dried for five hours. The dried resultant was pulverized with a pulverizer to obtain a coffee aroma-retaining powdery product. The product stably retains coffee aroma for a relatively long period of time and can be conveniently used such a manner of being dissolved in a hot water as a coffee beverage having satisfactory aroma and sweetness inherent to α,α-trehalose. It can be conveniently used as a raw material for beverage containing coffee and various confectioneries containing coffee.

Example C-9

Black tea aroma-retaining material

Fifty parts by weight of CTS crystalline pentahydrate, prepared by the method described in Example B-3, and 50 parts by weight of "TREHA®", a high-purity hydrous crystalline α,α-trehalose commercialized by Hayashibara Shoji, Inc., Okayama, Japan were mixed and dissolved in 50 parts by weight of water. The resulting solution was concentrated *in vacuo* to give a concentration of 90% and admixed with five parts by weight of a black tea commercialized by Mitsui Norin Co., Ltd., Tokyo, Japan, and spread on a plastic container, dried by passing through a dry and hot air, and solidified into a block. By pulverizing the resulting block with a pulverizer and drying the resultant, a powdery product was obtained. Since the product stably retains black tea aroma for a relatively long period of time, it can be conveniently used in the manner of being dissolved in a hot water and filtrating out the contained tea leaves to obtain a black tea beverage having a satisfactory aroma and sweetness inherent to α,α-trehalose.

Example C-10

Boiled-dried fish

Five parts by weight of a syrup, containing CTS and its saccharide derivative(s), prepared by the method described in Example B-1, was dissolved in 95 parts by weight of boiling water, and further boiled. Ten parts by weight of a raw anchovy were placed in a basket, soaked in the above solution, boiled, and then, taken out of the basket. By conventionally drying the boiled anchovy, the captioned product was obtained. Since the product sufficiently retains its boiled-dried fish aroma, it is useful as a soup stock having a satisfactory flavor.

Example C-11

Mirin powder

Three parts by weight of *mirin* were mixed with seven parts by weight of CTS crystalline anhydrate powder prepared by the method described in Example B-6, and the mixture was placed in a container and solidified into a block for two days while converting the CTS into CTS crystalline pentahydrate. By powdering the resulting block with a pulverizer and classifying, a flavorful aroma-retaining *mirin* was obtained. The product is useful as a seasoning for instant noodles or clean soups.

Example D-1

Juice powder

Thirty parts by weight of spray-dried grapefruit juice powder, 50 parts by weight of "SUNMALT-S®", a maltose commercialized by Hayashibara Shoji Inc., Okayama, Japan, 10 parts by weight of crystalline anhydrous maltitol, 10 parts by weight of the grapefruit aroma-retaining material prepared by the method described in Example C-6, 0.65 part by weight of anhydrous citric acid, 0.5 part by weight of pullulan, 0.1 part by weight of malic acid, 0.2 part by weight of "AA-2G®", a 2-O-α-glueosyl-L-ascorbic acid commercialized by Hayashibara Shoji, Inc., Okayama, Japan, and 0.1 part by weight of sodium citrate were mixed by stirring and pulverized. The resulting powder was granulated by a fluidized bed granulating machine with a blast temperature of 40°C, sprayed with an appropriate amount of 70% solution of "SUNMALT-S®", a maltose commercialized by Hayashibara Shoji, Inc., Okayama, Japan. After 30 min of granulation, the captioned product was obtained by weighing and packaging. The product contains about 30% of a juice powder. The product retains grapefruit aroma for a relatively long period of time free from foreign taste and odor, and it is a high quality juice powder.

Example D-2

Chewing gum

Three parts by weight of a gum base softened by heating and dissolving, two parts by weight of crystalline anhydrous maltitol, two parts by weight of xylitol, two parts by weight of "TREHA®", a high-purity hydrous crystalline α,α-trehalose commercialized by Hayashibara Shoji, Inc., Okayama, Japan, one part by weight of "SUNMALT-S®", a maltose commercialized by Hayashibara Shoji, Inc., Okayama, Japan, 0.5 part by weight of the aroma-retaining material containing menthol prepared by the method described in Example C-7, appropriate amounts of other aromatic substances and coloring agents were mixed and kneaded with a rolling machine. By shaping and packaging the resultant, the product was obtained. The product is a flavorful (texture, taste and smell) light calorie and anti-dental caries chewing gum retaining menthol aroma for a relatively long period of time.

Example D-3

Pullulam film

Two hundred parts by weight of "PULLULAN PI-20™" a pullulan commercialized by Hayashibara Shoji, Inc., Okayama, Japan, 20 parts by weight of the aroma-retaining material containing menthol prepared by the method described in Example C-7, 0.1 part by weight of "POLYPHENON™", a tea-extracted polyphenol commercialized by Mitsui Norin Co., Ltd., Tokyo, Japan, 0.05 part by weight of decanal, and 0.05 part by weight of sun yellow No.2A were mixed and dissolved in 750 parts by weight of water and then deaerated *in vacuo*. By spreading the resulting solution as a film material solution on polyethylene sheet uniformly and drying by passing through a hot air to obtain a 50°C, pullulan film 0.03mm in thickness. The transparent and glossy film, retaining menthol aroma for a long period of time and having stability against humidity change, can be used as foods or raw materials for secondary processings.

Example D-4

Bath salt

A bath salt was produced by mixing 90 parts by weight of a roast salt, 20 parts by weight of "TREHA®", a high-purity crystalline hydrous α,α-trehalose by commercialized by Hayashibara Shoji, Inc., Okayama, Japan, one part by weight of anhydrous silicic acid, two parts by weight of "AA-2G", an ascorbic acid 2-glucoside crystalline powder commercialized by Hayashibara Shoji, Inc., Okayama, Japan, two parts by weight of the aroma-retaining material containing grapefruit oil prepared by the method described in Example C-6, one part by weight of aroma-retaining material containing herb extract prepared by the method described in Example C-5, 0.5 part by weight of "αG HESPERIDIN", an α-glucosyl hesperidin commercialized by Hayashibara Shoji, Inc., Okayama, Japan. The product, retaining herb and grapefruit aroma even after preservation for a relatively long period of time, is usually used by diluting with the bath water by 100 to 10,000-folds as a high quality product having the effects of moisturizing, smoothing, and warming skins.

Example D-5

Cosmetic cream

Two parts by weight of polyoxyethylene glycol monostearate, five parts by weight of glycerin monostearate selfemulsifying, five parts by weight of "TREHA®", a high purity hydrous crystalline trehalose commercialized by Hayashibara Shoji, Inc., Okayama, Japan, five parts by weight of CTS crystalline pentahydrate prepared by the method described in Example B-3, one part by weight of "αG RUTIN", an α-glucosyl rutin commercialized by Hayashibara Co., Ltd., Okayama, Japan, one part by weight of "AA-2G®", an ascorbic acid 2-glucoside crystalline powder commercialized by Hayashibara Shoji, Inc., Okayama, Japan, one part by weight of liquid paraffin, 10 parts by weight of glycerin trioctanoate, and an appropriate amount of an antiseptic were mixed and dissolved by heating. To the resulting solution were added two parts by weight of L-lactic acid, five parts by weight of 1,3-butylene glycol and 66 parts by weight of purified water and emulsified with a homogenizer. Finally, by mixing the resulting emulsion with an appropriate amount of an aromatic substance, a cosmetic cream was produced. Since the product containing CTS retains the aroma and has an antioxidative effect, it is useful as a high-grade sunburn preventive agent for beauty skin, or whitening.

Example D-6

Shaped perfume

An incense tree extract was prepared by admixing 100 parts by weight of incense tree (sandalwood) with 1,000 parts by weight of 70%(w/v) ethanol aqueous solution, standing the mixture at 40°C for two hours, and filtrating the resultant. Five parts by weight of the above solution were diluted by fivefold with the above ethanol aqueous solution. To the solution was added 60 parts by weight of a "Tabunoki" (*Machilus thunbergii*) fine powder, 35 parts by weight of a cedar fine powder, six parts by weight of the CTS crystalline pentahydrate prepared by the method described in Example B-2, four parts by weight of a fresh preparation of α,α-trehalose as used in Example D-6, and one part by weight of pullulan used in Example D-3, and the resultant was kneaded with an appropriate amount of water in a usual manner to obtain a paste. The resulting paste was shaped with an oil pressure shaping machine 2mm in diameter, 136mm in length, and dried at room temperature (about 10 to 20°C) for three days to obtain a shaped perfume. The product is tough and high-grade shaped product because it retains sandalwood aroma and is hardly deteriorated, transformed, and broken.

Example E-1

Bactericide

One gram or 2g of an aroma-retaining material, prepared by mixing CTS crystalline anhydrate and ethanol prepared by the method described in Example C-1, was packaged in a porous paper bag. By packaging and sealing the resulting bag in an aluminium laminate bag, a bactericide was prepared. The product can be advantageously used as a sustained-release bacteriostat and/or bactericide which gradually releases ethanol when the contained paper bag is taken off. It is useful as a bacteriostat and/or bactericide for box lunch and side dish packaged in a relatively small space because the product only containing nontoxic ethanol and CTS is safe even when administered orally or attached skins or mucosae.

Test for bactericidal effect of the bactericide

A test for bactericidal effect of the aroma-retaining material consisting of CTS crystalline anhydrate and ethanol against some typical resident bacteria, i.e. *Bacillus subtilis* (ATCC6633), *Staphylococcus aureus* (ATCC6538), *Candida Albicans* (ATCC10231), and *Aspergillus niger* (ATCC16404) was carried out as follow: *Bacillus subtilis* (ATCC6633) and *Staphylococcus aureus* (ATCC6538) cultured in "NUTRIENT BROTH" commercialized by Difco Laboratories, USA, at 27°C for one day, and *Candida albicans* (ATCC10231) cultered in "YM BROTH" commercialized by Difco Laboratories, USA, at 27°C for one day were supplied as test solutions. *Aspergillus niger* (ATCC16404) cultured in a potato-dextrose agar medium commercialized by Difco Laboratories, USA, at 27°C for five days was suspended in the same medium as used in the above culture and then filtrated. The filtrate containing respectively spores was supplied as a test solution. 1.5 ml of each test solution was allowed to absorb unto an adsorbing pad (47mm in diameter; commercialized by Advantec Toyo Co., Ltd., Tokyo, Japan), and stood at 25°C for 30min. The adsorbing pads containing the bacteria were prepared. An aluminum laminate bags containing fresh bactericide and bactericide reserved at 25°C for 30 days were opened, and the fresh and reserved bactericide in porous paper bags were taken out. The porous paper

bags (containing 1g or 2g of the bactericide) were placed on glass dishes, and placed in a sealed container (having inner sizes of 30cm, 21cm and 9.5cm and an inner volume of about 5,985cm$^3$). Then the adsorbing pads containing bacteria were placed in the sealed container at the position of 10cm apart from each of the paper bags. After incubated at 25°C for 24 hours in the sealed container, viable cells were counted by extracting the bacteria from the adsorbing pads with physiological saline, appropriately diluting with the saline culturing on nutrient agar plates, and counting the formed colonies. The above results are shown only about the results of the bactericide freshly-prepared because preserved for 30 days one, and there were no difference in the bactericidal effects between the two bactericides. Therefore, the results with respect to the freshly-prepared bactericide are shown. As a control, the same experiment was carried out without ethanol, and the viable cells were counted. The data of viable cells, incubated with the bactericide for 24 hours, are in table 6.

Table 6

| Bacteria | Number of Viable Cells (Number)* | | | |
|---|---|---|---|---|
| | Starting | After 24 hours | | |
| | | bactericide (containing 1g) | bactericide (containing 2g) | without bactericide (Control) |
| *B. subtilis* (ATCC 6633) | $1.4 \times 10^4$ | $1.6 \times 10^4$ | 0 | $3.0 \times 10^7$ |
| *S. aureus* (ATCC 6538) | $1.4 \times 10^7$ | $2.0 \times 10^7$ | 0 | $4.1 \times 10^8$ |
| *C. albicans* (ATCC 10231) | $2.8 \times 10^7$ | $2.7 \times 10^7$ | 0 | $3.9 \times 10^7$ |
| *A. niger* (ATCC 16404) | $2.5 \times 10^3$ | $2.9 \times 10^3$ | 0 | $3.6 \times 10^3$ |

NOTE *: The number of viable cells per one adsorbing pad absorbing 1.5 ml of test solution.

As evident from the result in table 6, the bactericide, containing CTS in the form of a crystalline anhydrous, being packaged in the porous paper bag, and retaining ethanol as a bactericide, released ethanol and exerted bacteriostatic and/or bactericidal effect in a dose dependent manner. The bacteriostatic effect was revealed in the test using the paper bags containing 1g of the bactericide, which tended to inhibit the increasing number of bacteria. The bactericidal effect was revealed in the paper bags containing 2g of the bactericide, which completely killed the bacteria and/or the spore in the container having about 6L volume within 24 hours. The above result shows that the bactericide of the present invention has a satisfactory bacteriostatic and/or bacteriocidal effect.

INDUSTRIAL APPLICABILITY

As described above, according to the present invention, the aroma-retaining material having the effect of adsorbing and retaining aroma and being satisfactory stable can be obtained without affecting aroma or aroma-retaining material by mixing an aromatic substance and CTS or one or more members selected from the group consisting of syrup, mascuites, solid, and powder containing CTS and its saccharide derivative(s). It also enables to produce an aroma-retaining composition more easily. Since the aroma-retaining material of the present invention has retained-release property, it is not useful as only sustained-releasing agent for retaining aroma but also sustained-releasing bacteriostat and/or bactericide in case of the aromatic substance having a bactericidal effect. Thus, the present invention will give a great use on the industrial fields, for example, it provides a product having a satisfactory sustained aroma and stability.

"*Bacillus globisporus* C9" (FERM BP-7143), described in this specification, was deposited and accepted on April 25, 2000, in International Patent Organism Depositary National Institute of Advanced Industrial Science and Technology, Tsukuba Central 6, 1-1, Higashi 1-Chome Tsukuba-shi, Ibaraki-ken, 305-8566, Japan.

**Claims**

1. A method for retaining aroma **characterized in that** it comprises a step of incorporating a cyclic tetrasaccharide represented by Chemical Formula 1 or a mixture of the cycle tetrasaccharide and its saccharide derivative into an

aromatic substance or a composition comprising said aromatic substance:

Chemical formula 1:

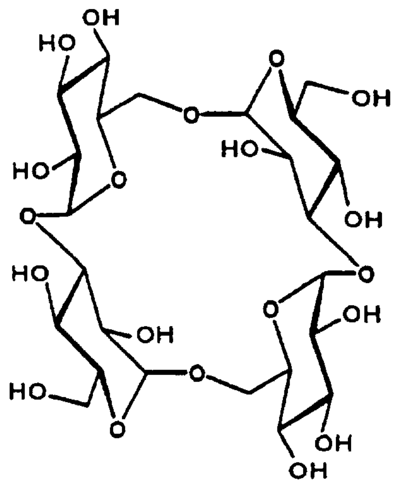

2. The method of claim 1, **characterized in that** wherein said aromatic substance is a natural aromatic substance derived from an animal or plant, a composition comprising said natural aromatic substance, or a synthetic aromatic substance.

3. The method of claim 1 or 2, **characterized in that** wherein said aromatic substance is ethanol and/or acetic acid.

4. The method of claim 1, 2, or 3, **characterized in that** wherein said cyclic tetrasaccharide or said mixture is added in an amount of 1 to 10,000 parts by weight to one part by weight of said aromatic substance on a dry solid basis of said cyclic tetrasaccharide.

5. The method of any one of claims 1 to 4, **characterized in that** wherein said cyclic tetrasaccharide or said mixture is in the form of a syrup, mascuite, solid, amorphous powder, hydrous crystalline powder, of anhydrous crystalline powder.

6. The method of any one of claims 1 to 5, **characterized in that** it further comprises a step of incorporating one or more members selected from the group consisting of monosaccharides, oligosaccharides, and polysaccharides with said cyclic tetrasaccharide or said mixture.

7. The method of any one of claims 1 to 6, **characterized in that** wherein said cyclic tetrasaccharide or said mixture is incorporated into said composition in the presence of water and/or an emulsifier.

8. The method of any one of claims 1 to 7, **characterized in that** wherein said cyclic tetrasaccharide or said mixture is produced from starch or phytoglycogen.

9. An aroma-retaining material, which is obtainable by any one of the methods of claims 1 to 8.

10. The aroma-retaining material of claim 9, **characterized in that** wherein said aromatic substance is in a composition comprising any one of alcohols, seasonings, and fermented foods.

11. The aroma-retaining material of claim 9, **characterized in that** wherein said aromatic substance is ethanol and/or acetic acid.

12. The aroma-retaining material of claim 9, 10 or 11, **characterized in that** it is in the form of a liquid, semisolid, solid, or powder.

13. The aroma-retaining material of any one of claims 9 to 12, **characterized in that** wherein said aromatic substance has a sustained-release property.

14. The aroma-retaining material of any one of claims 9 to 13, **characterized in that** it has a bacteriostat or bactericide effect.

15. A composition, which is prepared by co-existing or incorporating any one of said aroma-retaining materials of claims 9 to 14.

16. The composition of claim 15, **characterized in that** it is in the form of a food, cosmetic, pharmaceutical, or commodity.

17. An aroma-retaining agent, which comprises a cyclic tetrasaccharide represented by Chemical Formula 1 or a mixture of said cyclic tetrasaccharide and its saccharide derivative as effective ingredients.

18. A sustained-releasing agent having an aromatic substance, which comprises a cyclic tetrasaccharide represented by Chemical formula 1 or a mixture of said cyclic tetrasaccharide and its saccharide derivative.

19. A bacteriostat and/or bactericide, **characterized in that** it comprises ethanol and/or acetic acid, and a cyclic tetrasaccharide or a mixture of said cyclic tetrasaccharide and its saccharide derivative.

20. The bacteriostat and /or bactericide of claim 19, **characterized in that** it gradually releases ethanol and/or acetic acid.

21. A method for bacteriostasis and/or sterilization of foods, cosmetics, pharmaceuticals, or commodities, **characterized in that** said bacteriostat and/or bactericide of claim 19 or 20 are co-existed and/or incorporated into said foods, cosmetics, pharmaceuticals, or commodities.

## INTERNATIONAL SEARCH REPORT

International application No.
PCT/JP02/12196

A. CLASSIFICATION OF SUBJECT MATTER
Int.Cl[7] C11B9/00, A01N25/22, A61K7/00, A61K47/26, A61K47/36, A23L1/22

According to International Patent Classification (IPC) or to both national classification and IPC

B. FIELDS SEARCHED

Minimum documentation searched (classification system followed by classification symbols)
Int.Cl[7] C11B9/00, A01N25/22, A61K7/00, A61K47/26, A61K47/36, A23L1/22

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | US 5889179 A (THE UNITED STATES OF AMERICA AS REPRESENTED BY THE SECRETARY OF AGRICULTURE), 30 March, 1999 (30.03.99), (Family: none) | 1-7,9-21<br>8 |
| Y | WO 00/76472 A1 (THE PROCTER & GAMBLE CO.), 21 December, 2000 (21.12.00), & JP 2003-501456 A & US 6110449 A & EP 1185240 A1 | 1,2,4,5,7, 9,15-18 |
| Y | JP 6-189737 A (Sanchu Wasabi Kabushiki Kaisha), 12 July, 1994 (12.07.94), Claim 1; Par. No. [0006] (Family: none) | 3,10-14, 19-21 |

[X] Further documents are listed in the continuation of Box C.  [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 February, 2003 (25.02.03) | 11 March, 2003 (11.03.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/12196

C (Continuation). DOCUMENTS CONSIDERED TO BE RELEVANT

| Category* | Citation of document, with indication, where appropriate, of the relevant passages | Relevant to claim No. |
|---|---|---|
| Y | JP 63-181950 A (Yasushi TAKAHASHI), 27 July, 1988 (27.07.88), Claims; page 1, lower right column, lines 1 to 6 (Family: none) | 3,10-14, 19-21 |
| Y | JP 2001-48765 A (Kabushiki Kaisha Hayashibara Arts Mint), 20 February, 2001 (20.02.01), Claims (Family: none) | 6 |
| A | JP 7-316204 A (Toshiro NAKAGAWA), 05 December, 1995 (05.12.95), Claims; Par. No. [0002] (Family: none) | 1-21 |

Form PCT/ISA/210 (continuation of second sheet) (July 1998)